# EUROPEAN PATENT APPLICATION

(11) **EP 1 020 527 A1**
(43) Date of publication of application: **19.07.2000**
(21) Application number: 99933237.2
(22) Date of filing: 03.08.1999
(51) Int. Cl.: C12N 15/55

(54) **MUTATED BARNASE GENE AND PLANT TRANSFORMED BY THE SAME**

(30) Priority: 04.08.1998 JP 22006098
(71) Applicant: Japan Tobacco Inc., Tokyo 105-8422 (JP)
(72) Inventor: HAMADA, Kazuyuki, Japan Tobacco Inc., Shizuoaka 438-802 (JP); NAKAKIDO, Fumio, Japan Tobacco Inc., Shizuoka 438-0802 (JP)
(74) Representative: Reinhard - Skuhra - Weise & Partner
(86) International application number: JP9904167
(87) International publication number: WO0008176

(57) **Abstract**

A male sterile plant which is free from any undesirable character is constructed by expressing a barnase gene alone specifically in the anther. The barnase gene, which has been depressed by mutagenesis while sustaining its activity, is transferred into a plant and then expressed specifically in the anther to thereby efficiently give the aimed male sterile transformant.

SEQUENCE LISTING

<110> Japan Tobacco Inc.

<120> MUTANT BARNASE GENE AND TRANSGENIC PLANT TRANSFORMED BY SAID GENE

<130> 980687

<160> 7

<210> 1
<211> 343
<212> DNA
<213> Bacillus amyloliquefaciens
<220>
<221> NAME/KEY: CDS
<222> 1..336

<400> 1
atg gta ccg gtt atc aac acg ttt gac ggg gtt gcg gat tat ctt cag 48
Met Val Pro Val Ile Asn Thr Phe Asp Gly Val Ala Asp Tyr Leu Gln
1               5                   10                  15
aca tat cat aag cta cct gat aat tac att aca aaa tca gaa gca caa 96
Thr Tyr His Lys Leu Pro Asp Asn Tyr Ile Thr Lys Ser Glu Ala Gln
            20                  25                  30
gcc ctc ggc tgg gtg gca tca aaa ggg aac ctt gca gac gtc gct ccg 144
Ala Leu Gly Trp Val Ala Ser Lys Gly Asn Leu Ala Asp Val Ala Pro
        35                  40                  45
ggg aaa agc atc ggc gga gac atc ttc tca aac agg gaa ggc aaa ctc 192
Gly Lys Ser Ile Gly Gly Asp Ile Phe Ser Asn Arg Glu Gly Lys Leu

    50                  55                  60

ccg ggc aaa agc gga cga aca tgg cgt gaa gcg gat att aac tat aca 240

Pro Gly Lys Ser Gly Arg Thr Trp Arg Glu Ala Asp Ile Asn Tyr Thr

65                  70                  75                  80

tca ggc ttc aga aat tca gac cgg att ctt tac tca agc gac tgg ctg 288

Ser Gly Phe Arg Asn Ser Asp Arg Ile Leu Tyr Ser Ser Asp Trp Leu

                85                  90                  95

att tac aaa aca acg gac cat tat cag acc ttt aca aaa atc aga taa 336

Ile Tyr Lys Thr Thr Asp His Tyr Gln Thr Phe Thr Lys Ile Arg

            100                 105                 110

ggtaacc 343

<210> 2

<211> 112

<212> PRT

<213> Bacillus amyloliquefaciens

<400> 2

Met Val Pro Val Ile Asn Thr Phe Asp Gly Val Ala Asp Tyr Leu Gln

1               5                   10                  15

Thr Tyr His Lys Leu Pro Asp Asn Tyr Ile Thr Lys Ser Glu Ala Gln

            20                  25                  30

Ala Leu Gly Trp Val Ala Ser Lys Gly Asn Leu Ala Asp Val Ala Pro

        35                  40                  45

Gly Lys Ser Ile Gly Gly Asp Ile Phe Ser Asn Arg Glu Gly Lys Leu

    50                  55                  60

Pro Gly Lys Ser Gly Arg Thr Trp Arg Glu Ala Asp Ile Asn Tyr Thr

65                  70                  75                  80

Ser Gly Phe Arg Asn Ser Asp Arg Ile Leu Tyr Ser Ser Asp Trp Leu

85                  90                  95

Ile Tyr Lys Thr Thr Asp His Tyr Gln Thr Phe Thr Lys Ile Arg

100                 105                 110

<210>  3

<211>  342

<212>  DNA

<213>

<400>  3

atggtaccgg ttattcaaca cgtttgacgg ggttgcggat tatcttcaga catatcataa      60

gctacctgat aattacatta caaaatcaga agcacaagcc ctcggctggg tggcatcaaa     120

agggaacctt gcagacgtcg ctccggggaa aagcatcggc ggagacatct tctcaaacag     180

ggaaggcaaa ctcccgggca aaagcggacg aacatggcgt gaagcggata ttaactatac     240

atcaggcttc agaaattcag accggattct ttactcaagc gactggctga tttacaaaac     300

aacggaccat tatcagacct ttacaaaaat cagtaatcta ga                        342

<210>  4

<211>  6548

<212>  DNA

<213> Escherichia coli LE392

<220>

<223>  Clone: pTS172

<400>  4

aattcaagct tgacgtcagg tggcactttt cggggaaatg tgcgcggaac ccctatttgt      60

ttatttttct aaatacattc aaatatgtat ccgctcatga gacaataacc ctgataaatg     120

cttcaataat attgaaaaag gaagagtatg agtattcaac atttccgtgt cgcccttatt     180

cccttttttg cggcattttg ccttcctgtt tttgctcacc cagaaacgct ggtgaaagta     240

aaagatgctg aagatcagtt gggtgcacga gtgggttaca tcgaactgga tctcaacagc 300
ggtaagatcc ttgagagttt tcgccccgaa gaacgttttc caatgatgag cacttttaaa 360
gttctgctat gtggcgcggt attatcccgt attgacgccg ggcaagagca actcggtcgc 420
cgcatacact attctcagaa tgacttggtt gagtactcac cagtcacaga aaagcatctt 480
acggatggca tgacagtaag agaattatgc agtgctgcca taaccatgag tgataacact 540
gcggccaact tacttctgac aacgatcgga ggaccgaagg agctaaccgc ttttttgcac 600
aacatggggg atcatgtaac tcgccttgat cgttgggaac cggagctgaa tgaagccata 660
ccaaacgacg agcgtgacac cacgatgcct gtagcaatgg caacaacgtt gcgcaaacta 720
ttaactggcg aactacttac tctagcttcc cggcaacaat taatagactg gatggaggcg 780
gataaagttg caggaccact tctgcgctcg gcccttccgg ctggctggtt tattgctgat 840
aaatctggag ccggtgagcg tgggtctcgc ggtatcattg cagcactggg gccagatggt 900
aagccctccc gtatcgtagt tatctacacg acggggagtc aggcaactat ggatgaacga 960
aatagacaga tcgctgagat aggtgcctca ctgattaagc attggtaact gtcagaccaa 1020
gtttactcat atatacttta gattgattta aaacttcatt tttaatttaa aaggatctag 1080
gtgaagatcc tttttggctc gagtctcatg accaaaatcc cttaacgtga gttttcgttc 1140
cactgagcgt cagaccccgt agaaaagatc aaaggatctt cttgagatcc tttttttctg 1200
cgcgtaatct gctgcttgca aacaaaaaaa ccaccgctac cagcggtggt ttgtttgccg 1260
gatcaagagc taccaactct ttttccgaag gtaactggct tcagcagagc gcagatacca 1320
aatactgtcc ttctagtgta gccgtagtta ggccaccact tcaagaactc tgtagcaccg 1380
cctacatacc tcgctctgct aatcctgtta ccagtggctg ctgccagtgg cgataagtcg 1440
tgtcttaccg ggttggactc aagacgatag ttaccggata aggcgcagcg gtcgggctga 1500
acggggggtt cgtgcacaca gcccagcttg gagcgaacga cctacaccga actgagatac 1560
ctacagcgtg agcattgaga aagcgccacg cttcccgaag ggagaaaggc ggacaggtat 1620
ccggtaagcg gcagggtcgg aacaggagag cgcacgaggg agcttccagg gggaaacgcc 1680
tggtatcttt atagtcctgt cgggtttcgc cacctctgac ttgagcgtcg atttttgtga 1740
tgctcgtcag gggggcggag cctatggaaa aacgccagca acgcggcctt tttacggttc 1800
ctggcctttt gctggccttt tgctcacatg ttctttcctg cgttatcccc tgattctgtg 1860
gataaccgta ttaccgcctt tgagtgagct gataccgctc gccgcagccg aacgaccgag 1920
cgcagcgagt cagtgagcga ggaagcggaa gagcgcccaa tacgcaaacc gcctctcccc 1980

gcgcgttggc ctgatcagaa ttcatatgca cgtgttcccg atctagtaac atagatgaca 2040
ccgcgcgcga taatttatcc tagtttgcgc gctatatttt gttttctatc gcgtattaaa 2100
tgtataattg cgggactcta atcataaaaa cccatctcat aaataacgtc atgcattaca 2160
tgttaattat tacatgctta acgtaattca acagaaatta tatgataatc atcgcaagac 2220
cggcaacagg attcaatctt aagaaacttt attgccaaat gtttgaacga tctgcttcgg 2280
aggttacctt atctgatttt tgtaaaggtc tgataatggt ccgttgtttt gtaaatcagc 2340
cagtcgcttg agtaaagaat ccggtctgaa tttctgaagc ctgatgtata gttaatatcc 2400
gcttcacgcc atgttcgtcc gcttttgccc gggagtttgc cttccctgtt tgagaagatg 2460
tctccgccga tgcttttccc cggagcgacg tctgcaaggt tcccttttga tgccacccag 2520
ccgagggctt gtgcttctga ttttgtaatg taattatcag gtagcttatg atatgtctga 2580
agataatccg caaccccgtc aaacgtgttg ataaccggta ccatcgcgac ggcttgatgg 2640
atctcttgct ggacaccggg atgctaggat gggttatcgt ggccggcgtg cgtgtgtggc 2700
ttttgtaggc gccggcgacg gcgggggcaa tgtggcaggt gagtcacggt gcaagcgtgc 2760
gcaagtgact gcaacaacca aggacggtca tggcgaaagc acctcacgcg tccaccgtct 2820
acaggatgta gcagtagcac ggtgaaagaa gtgttgtccc gtccattagg tgcattctca 2880
ccgttggcca gaacaggacc gttcaacagt taggttgagt gtaggacttt tacgtggtta 2940
atgtatggca aatagtagta aattttgccc ccattggtct ggctgagata gaacatattc 3000
tggaaagcct ctagcatatc ttttttgaca gctaaacttt gcttcttgcc ttcttggtct 3060
agcaatgacg ttgcccatgt cgtggcaaac atctggtaag gtaactgtat tcgtttgttc 3120
ccttcaacgg ctcaatcccc acaggccaag ctatcctttc cttggcagta taggctcctt 3180
gagagattat actaccattt ttaagtgctt ataaagacga tgctctctaa ccagatcgat 3240
cagaaacaca aagttttagc agcgtaatat cccacacaca tacacacacg aagctatgcc 3300
tcctcatttt ccgagagatt ctgacagtga ccagaatgtc agaatgccat ttcatgggca 3360
caagtcgatc cacaagcttc ttggtggagg tcaaggtgtg ctattattat tcgctttcta 3420
ggaaattatt cagaattagt gccttttatc ataacttctc tctgagccga tgtggttttg 3480
gatttcattg ttgggagcta tgcagttgcg gatattctgc tgtggaagaa caggaactta 3540
tctgcggggg tccttgctgg ggcaacattg atatggttcc tgttcgatgt agtagaatac 3600
aatataattc cgctcctttg ccagattgcc attcttgcca tgcttgtgat cttcatttgg 3660
tcaaatgccg caccactctt ggacaggtat tagctttatt tcctgtggag atggtagaaa 3720

actcagctta cagaaatggc atttcacgta gtataacgca agacattagg tactaaaact 3780
caactaactg tttccgaatt tcagggcccc tccaaggatc ccagaaatca tcatctctga 3840
acatgccttc agagaaatgg cattgaccgt ccattacaaa ctaacgtaca ctgtatctgt 3900
tctttacgac attgcatgtg gaaaggatct gaagagattt ctcctggtac ataataatct 3960
actcctttgc tacgttaata agagatgtaa aaacatgcaa cagttccagt gccaacattg 4020
tccaaggatt gtgcaattct ttctggagcg ctaaaattga ccagattaga cgcatcagaa 4080
tattgaattg cagagttagc caataatcct cataatgtta atgtgctatt gttgttcact 4140
actcaatata gttctggact aacaatcaga ttgtttatga tattaaggtg gttggatctc 4200
tattggtatt gtcggcgatt ggaagttctt gcagcttgac aagtctacta tatattggta 4260
ggtattccag ataaatatta aattttaata aaacaatcac acagaaggat ctgcggccgc 4320
tagcctaggc ccgggcccac aaaaatctga gcttaacagc acagttgctc ctctcagagc 4380
agaatcgggt attcaacacc ctcatatcaa ctactacgtt gtgtataacg gtccacatgc 4440
cggtatatac gatgactggg gttgtacaaa ggcggcaaca aacggcgttc ccggagttgc 4500
acacaagaaa tttgccacta ttacagaggc aagagcagca gctgacgcgt acacaacaag 4560
tcagcaaaca gacaggttga acttcatccc caaaggagaa gctcaactca agcccaagag 4620
ctttgctaag gccctaacaa gcccaccaaa gcaaaaagcc cactggctca cgctaggaac 4680
caaaaggccc agcagtgatc cagccccaaa agagatctcc tttgccccgg agattacaat 4740
ggacgatttc ctctatcttt acgatctagg aaggaagttc gaaggtgaag gtgacgacac 4800
tatgttcacc actgataatg agaaggttag cctcttcaat ttcagaaaga atgctgaccc 4860
acagatggtt agagaggcct acgcagcagg tctcatcaag acgatctacc cgagtaacaa 4920
tctccaggag atcaaatacc ttcccaagaa ggttaaagat gcagtcaaaa gattcaggac 4980
taattgcatc aagaacacag agaaagacat atttctcaag atcagaagta ctattccagt 5040
atggacgatt caaggcttgc ttcataaacc aaggcaagta atagagattg gagtctctaa 5100
aaaggtagtt cctactgaat ctaaggccat gcatggagtc taagattcaa atcgaggatc 5160
taacagaact cgccgtgaag actggcgaac agttcataca gagtctttta cgactcaatg 5220
acaagaagaa aatcttcgtc aacatggtgg agcacgacac tctggtctac tccaaaaatg 5280
tcaaagatac agtctcagaa gaccaaaggg ctattgagac ttttcaacaa aggataattt 5340
cgggaaacct cctcggattc cattgcccag ctatctgtca cttcatcgaa aggacagtag 5400
aaaaggaagg tggctcctac aaatgccatc attgcgataa aggaaaggct atcattcaag 5460

atgcctctgc cgacagtggt cccaaagatg gacccccacc cacgaggagc atcgtggaaa 5520

aagaagacgt tccaaccacg tcttcaaagc aagtggattg atgtgacatc tccactgacg 5580

taagggatga cgcacaatcc cactatcctt cgcaagaccc ttcctctata taaggaagtt 5640

catttcattt ggagaggaca cgctgaaatc accagtctct ctctataaat ctatctctct 5700

ctctataacc atggacccag aacgacgccc ggccgacatc cgccgtgcca ccgaggcgga 5760

catgccggcg gtctgcacca tcgtcaacca ctacatcgag acaagcacgg tcaacttccg 5820

taccgagccg caggaaccgc aggagtggac ggacgacctc gtccgtctgc gggagcgcta 5880

tccctggctc gtcgccgagg tggacggcga ggtcgccggc atcgcctacg cgggcccctg 5940

gaaggcacgc aacgcctacg actggacggc cgagtcgacc gtgtacgtct ccccccgcca 6000

ccagcggacg ggactgggct ccacgctcta cacccacctg ctgaagtccc tggaggcaca 6060

gggcttcaag agcgtggtcg ctgtcatcgg gctgcccaac gacccgagcg tgcgcatgca 6120

cgaggcgctc ggatatgccc cccgcggcat gctgcgggcg gccggcttca agcacgggaa 6180

ctggcatgac gtgggtttct ggcagctgga cttcagcctg ccggtaccgc cccgtccggt 6240

cctgcccgtc accgagatct gagatcacgc gttctaggat cccccgatga gctaagctag 6300

ctatatcatc aatttatgta ttacacataa tatcgcactc agtctttcat ctacggcaat 6360

gtaccagctg atataatcag ttattgaaat atttctgaat ttaaacttgc atcaataaat 6420

ttatgttttt gcttggacta taatacctga cttgttattt tatcaataaa tatttaaact 6480

atatttcttt caagatggga attaacatct acaaattgcc ttttcttatc gaccatgtac 6540

gtatcgcg 6548

<210> 5

<211> 6539

<212> DNA

<213> Escherichia coli LE392

<220>

<223> Clone: pTS431

<400> 5

aattcaagct tgacgtcagg tggcactttt cggggaaatg tgcgcggaac ccctatttgt 60

ttatttttct aaatacattc aaatatgtat ccgctcatga gacaataacc ctgataaatg 120
cttcaataat attgaaaaag gaagagtatg agtattcaac atttccgtgt cgcccttatt 180
cccttttttg cggcattttg ccttcctgtt tttgctcacc cagaaacgct ggtgaaagta 240
aaagatgctg aagatcagtt gggtgcacga gtgggttaca tcgaactgga tctcaacagc 300
ggtaagatcc ttgagagttt tcgccccgaa gaacgttttc caatgatgag cacttttaaa 360
gttctgctat gtggcgcggt attatcccgt attgacgccg ggcaagagca actcggtcgc 420
cgcatacact attctcagaa tgacttggtt gagtactcac cagtcacaga aaagcatctt 480
acggatggca tgacagtaag agaattatgc agtgctgcca taaccatgag tgataacact 540
gcggccaact tacttctgac aacgatcgga ggaccgaagg agctaaccgc ttttttgcac 600
aacatggggg atcatgtaac tcgccttgat cgttgggaac cggagctgaa tgaagccata 660
ccaaacgacg agcgtgacac cacgatgcct gtagcaatgg caacaacgtt gcgcaaacta 720
ttaactggcg aactacttac tctagcttcc cggcaacaat taatagactg gatggaggcg 780
gataaagttg caggaccact tctgcgctcg gcccttccgg ctggctggtt tattgctgat 840
aaatctggag ccggtgagcg tgggtctcgc ggtatcattg cagcactggg gccagatggt 900
aagccctccc gtatcgtagt tatctacacg acggggagtc aggcaactat ggatgaacga 960
aatagacaga tcgctgagat aggtgcctca ctgattaagc attggtaact gtcagaccaa 1020
gtttactcat atatacttta gattgattta aaacttcatt tttaatttaa aaggatctag 1080
gtgaagatcc tttttggctc gagtctcatg accaaaatcc cttaacgtga gttttcgttc 1140
cactgagcgt cagaccccgt agaaaagatc aaaggatctt cttgagatcc tttttttctg 1200
cgcgtaatct gctgcttgca aacaaaaaaa ccaccgctac cagcggtggt ttgtttgccg 1260
gatcaagagc taccaactct ttttccgaag gtaactggct tcagcagagc gcagatacca 1320
aatactgtcc ttctagtgta gccgtagtta ggccaccact tcaagaactc tgtagcaccg 1380
cctacatacc tcgctctgct aatcctgtta ccagtggctg ctgccagtgg cgataagtcg 1440
tgtcttaccg ggttggactc aagacgatag ttaccggata aggcgcagcg gtcgggctga 1500
acggggggtt cgtgcacaca gcccagcttg gagcgaacga cctacaccga actgagatac 1560
ctacagcgtg agcattgaga aagcgccacg cttcccgaag ggagaaaggc ggacaggtat 1620
ccggtaagcg gcagggtcgg aacaggagag cgcacgaggg agcttccagg gggaaacgcc 1680
tggtatcttt atagtcctgt cgggtttcgc cacctctgac ttgagcgtcg atttttgtga 1740
tgctcgtcag gggggcggag cctatggaaa aacgccagca acgcggcctt tttacggttc 1800

ctggcctttt gctggccttt tgctcacatg ttctttcctg cgttatcccc tgattctgtg 1860
gataaccgta ttaccgcctt tgagtgagct gataccgctc gccgcagccg aacgaccgag 1920
cgcagcgagt cagtgagcga ggaagcggaa gagcgcccaa tacgcaaacc gcctctcccc 1980
gcgcgttggc ctgatcagaa ttcttcccga tctagtaaca tagatgacac cgcgcgcgat 2040
aatttatcct agtttgcgcg ctatattttg ttttctatcg cgtattaaat gtataattgc 2100
gggactctaa tcataaaaac ccatctcata aataacgtca tgcattacat gttaattatt 2160
acatgcttaa cgtaattcaa cagaaattat atgataatca tcgcaagacc ggcaacagga 2220
ttcaatctta agaaacttta ttgccaaatg tttgaacgat ctgcttcgga tcctctagat 2280
tactgatttt tgtaaaggtc tgataatggt ccgttgtttt gtaaatcagc cagtcgcttg 2340
agtaaagaat ccggtctgaa tttctgaagc ctgatgtata gttaatatcc gcttcacgcc 2400
atgttcgtcc gcttttgccc gggagtttgc cttccctgtt tgagaagatg tctccgccga 2460
tgcttttccc cggagcgacg tctgcaaggt tcccttttga tgccacccag ccgagggctt 2520
gtgcttctga ttttgtaatg taattatcag gtagcttatg atatgtctga agataatccg 2580
caaccccgtc aaacgtgttg aataaccggt accatcgcga cggcttgatg gatctcttgc 2640
tggacaccgg gatgctagga tgggttatcg tggccggcgt gcgtgtgtgg cttttgtagg 2700
cgccggcgac ggcgggggca atgtggcagg tgagtcacgg tgcaagcgtg cgcaagtgac 2760
tgcaacaacc aaggacggtc atggcgaaag cacctcacgc gtccaccgtc tacaggatgt 2820
agcagtagca cggtgaaaga agtgttgtcc cgtccattag gtgcattctc accgttggcc 2880
agaacaggac cgttcaacag ttaggttgag tgtaggactt ttacgtggtt aatgtatggc 2940
aaatagtagt aaattttgcc cccattggtc tggctgagat agaacatatt ctggaaagcc 3000
tctagcatat cttttttgac agctaaactt tgcttcttgc cttcttggtc tagcaatgac 3060
gttgcccatg tcgtggcaaa catctggtaa ggtaactgta ttcgtttgtt cccttcaacg 3120
gctcaatccc cacaggccaa gctatccttt ccttggcagt ataggctcct tgagagatta 3180
tactaccatt tttaagtgct tataaagacg atgctctcta accagatcga tcagaaacac 3240
aaagttttag cagcgtaata tcccacacac atacacacac gaagctatgc ctcctcattt 3300
tccgagagat tctgacagtg accagaatgt cagaatgcca tttcatgggc acaagtcgat 3360
ccacaagctt cttggtggag gtcaaggtgt gctattatta ttcgctttct aggaaattat 3420
tcagaattag tgccttttat cataacttct ctctgagccg atgtggtttt ggatttcatt 3480
gttgggagct atgcagttgc ggatattctg ctgtggaaga acaggaactt atctgcgggg 3540

gtccttgctg gggcaacatt gatatggttc ctgttcgatg tagtagaata caatataatt 3600
ccgctccttt gccagattgc cattcttgcc atgcttgtga tcttcatttg gtcaaatgcc 3660
gcaccactct tggacaggta ttagctttat ttcctgtgga gatggtagaa aactcagctt 3720
acagaaatgg catttcacgt agtataacgc aagacattag gtactaaaac tcaactaact 3780
gtttccgaat ttcagggccc ctccaaggat cccagaaatc atcatctctg aacatgcctt 3840
cagagaaatg gcattgaccg tccattacaa actaacgtac actgtatctg ttctttacga 3900
cattgcatgt ggaaaggatc tgaagagatt tctcctggta cataataatc tactcctttg 3960
ctacgttaat aagagatgta aaaacatgca acagttccag tgccaacatt gtccaaggat 4020
tgtgcaattc tttctggagc gctaaaattg accagattag acgcatcaga atattgaatt 4080
gcagagttag ccaataatcc tcataatgtt aatgtgctat tgttgttcac tactcaatat 4140
agttctggac taacaatcag attgtttatg atattaaggt ggttggatct ctattggtat 4200
tgtcggcgat tggaagttct tgcagcttga caagtctact atatattggt aggtattcca 4260
gataaatatt aaattttaat aaaacaatca cacagaagga tctgcggccg ctagcctagg 4320
cccgggccca caaaaatctg agcttaacag cacagttgct cctctcagag cagaatcggg 4380
tattcaacac cctcatatca actactacgt tgtgtataac ggtccacatg ccggtatata 4440
cgatgactgg ggttgtacaa aggcggcaac aaacggcgtt cccggagttg cacacaagaa 4500
atttgccact attacagagg caagagcagc agctgacgcg tacacaacaa gtcagcaaac 4560
agacaggttg aacttcatcc ccaaaggaga agctcaactc aagcccaaga gctttgctaa 4620
ggccctaaca agcccaccaa agcaaaaagc ccactggctc acgctaggaa ccaaaaggcc 4680
cagcagtgat ccagccccaa aagagatctc ctttgccccg gagattacaa tggacgattt 4740
cctctatctt tacgatctag gaaggaagtt cgaaggtgaa ggtgacgaca ctatgttcac 4800
cactgataat gagaaggtta gcctcttcaa tttcagaaag aatgctgacc cacagatggt 4860
tagagaggcc tacgcagcag gtctcatcaa gacgatctac ccgagtaaca atctccagga 4920
gatcaaatac cttcccaaga aggttaaaga tgcagtcaaa agattcagga ctaattgcat 4980
caagaacaca gagaaagaca tatttctcaa gatcagaagt actattccag tatggacgat 5040
tcaaggcttg cttcataaac caaggcaagt aatagagatt ggagtctcta aaaaggtagt 5100
tcctactgaa tctaaggcca tgcatggagt ctaagattca aatcgaggat ctaacagaac 5160
tcgccgtgaa gactggcgaa cagttcatac agagtctttt acgactcaat gacaagaaga 5220
aaatcttcgt caacatggtg gagcacgaca ctctggtcta ctccaaaaat gtcaaagata 5280

cagtctcaga agaccaaagg gctattgaga cttttcaaca aaggataatt tcgggaaacc 5340
tcctcggatt ccattgccca gctatctgtc acttcatcga aaggacagta gaaaaggaag 5400
gtggctccta caaatgccat cattgcgata aaggaaaggc tatcattcaa gatgcctctg 5460
ccgacagtgg tcccaaagat ggacccccac ccacgaggag catcgtggaa aaagaagacg 5520
ttccaaccac gtcttcaaag caagtggatt gatgtgacat ctccactgac gtaagggatg 5580
acgcacaatc ccactatcct tcgcaagacc cttcctctat ataaggaagt tcatttcatt 5640
tggagaggac acgctgaaat caccagtctc tctctataaa tctatctctc tctctataac 5700
catggaccca gaacgacgcc cggccgacat ccgccgtgcc accgaggcgg acatgccggc 5760
ggtctgcacc atcgtcaacc actacatcga gacaagcacg gtcaacttcc gtaccgagcc 5820
gcaggaaccg caggagtgga cggacgacct cgtccgtctg cgggagcgct atccctggct 5880
cgtcgccgag gtggacggcg aggtcgccgg catcgcctac gcgggcccct ggaaggcacg 5940
caacgcctac gactggacgg ccgagtcgac cgtgtacgtc tccccccgcc accagcggac 6000
gggactgggc tccacgctct acacccacct gctgaagtcc ctggaggcac agggcttcaa 6060
gagcgtggtc gctgtcatcg ggctgcccaa cgacccgagc gtgcgcatgc acgaggcgct 6120
cggatatgcc ccccgcggca tgctgcgggc ggccggcttc aagcacggga actggcatga 6180
cgtgggtttc tggcagctgg acttcagcct gccggtaccg ccccgtccgg tcctgcccgt 6240
caccgagatc tgagatcacg cgttctagga tcccccgatg agctaagcta gctatatcat 6300
caatttatgt attacacata atatcgcact cagtctttca tctacggcaa tgtaccagct 6360
gatataatca gttattgaaa tatttctgaa tttaaacttg catcaataaa tttatgtttt 6420
tgcttggact ataatacctg acttgttatt ttatcaataa atatttaaac tatatttctt 6480
tcaagatggg aattaacatc tacaaattgc cttttcttat cgaccatgta cgtatcgcg 6539

<210> 6
<211> 37
<212> DNA
<213> Artificial Sequence
<220>
<223> Primer 1

<400> 6
cgttcggctc gatggtaccg gttatcaaca cgtttga 37

<210> 7
<211> 38
<212> DNA
<213> Artificial Sequence
<220>
<223> Primer 2

<400> 7
cctctagatt atctgatttt tgtaaaggtc tgataatg 38

## Description

The present application claims priority from Japanese Patent Application No.: Hei 10-220060, the disclosure of which is incorporated herein by reference.

### FIELD OF THE INVENTION

This invention relates to a mutant barnase gene which makes it possible to efficiently yield a male sterile transgenic plant when expressed in a specific site of a plant, in particular, anther-specifically. The present invention further relates to a recombinant vector capable of expressing the mutant barnase gene of the invention in host cells, a plant transformed by this vector and a method for constructing a transgenic plant.

### PRIOR ART

Barnase is an RNase originating in *Bacillus amyloliquefaciens* (S. Nishimura and M. Nomura, Biochem. Biophys. Acta 30, 430-431:1958; R.W. Hartley, J. Mol. Biol., 202, 913-915:1988). This enzyme has 110 amino acid residues and hydrolyzes RNA. When expressed in cells, this enzyme inhibits the functions of the cells as a result of its potent RNase activity and thus causes cell death in many cases. By using this characteristic, it is therefore expected that the function of the specific site can be selectively controlled by expressing the barnase gene in a specific site of a plant.

PCT International Publication WO89/10396 discloses a technique whereby a male sterile plant is obtained by constructing a male sterility gene by ligating the above-described barnase gene to the downstream of an anther tapetal cell-specific expression promoter and introducing the thus obtained gene into a plant. Male sterilization techniques are of great value in efficiently developing an F1 hybrid variety.

When the barnase gene is employed as a male sterility gene, however, it is frequently observed that resulting male sterile transgenic plants exhibit unfavorable characteristics. PCT International Publication WO96/26283 refers to this problem in rice. It is also reported that similar phenomena are observed not only in rice but in lettuce (Scientia Horticulturae 55, 125-139:1993; Arlette Reymaerts, Hilde Van de Wiele, Greta De Sutter, Jan Janssens: Engineered genes for fertility control and their application in hybrid seed production). According to this report, a plant with depressed activity was constructed by introducing a male sterility gene comprising a tobacco anther-specific promoter (TA29) and a barnase gene into lettuce.

Although reasons for these phenomena have not been accurately clarified so far, it is assumed that the so-called "position effect" of the site in which the gene is transferred may account for the mechanism (PCT International Publication WO96/26283). More specifically, a desired male sterile plant should be constructed if the male sterility gene is expressed exclusively in the target site (i.e., anthers). However, there is a possibility that the barnase might be expressed also in tissues other than anthers although in a very small dose under the action of expression regulators (for example, an endogenous enhancer) existing in the vicinity of the gene transfer site. In such a case, the unfavorable characteristics as described above can appear because barnase has a strong enzymatic activity.

To overcome this problem, the method disclosed in PCT International Publication WO96/26283 exploits the character of cauliflower mosaic virus 35S promoter (hereinafter referred to as CaMV35S promoter) of being expressed potently in tissues other than anthers. Namely, barstar, i.e., an inhibitory protein against barnase is employed therein. The barstar gene ligated to a CaMV35S promoter is transferred into a plant simultaneously with a barnase gene and then the barstar gene is constitutively expressed in tissues other than the anthers, thereby eliminating the effects of the barnase in tissues other than the anthers. However, it is necessary in this method to transfer not only a barnase gene but also a barstar gene, and hence, the problem is that the application of this technique to the breeding of F1 varieties of rice or corn seeds may give rise to a gene silencing. "Gene silencing" is a phenomenon wherein the expression of a gene is inhibited when plural copies of the foreign gene are introduced. It is known that this problem frequently occurs when the expression of a foreign gene is effected by a 35S promoter, though the detailed mechanism thereof has not been clarified yet (R.B. Flavel, Proc. Natl. Acad. Sci. USA 91, 3490-3496:1994; J. Finnegan, Bio. Technology 12, 883-888:1994; M.A. Matzke and A.J.M. Matzke, Plant Physiol., 107, 679-685:1995). Since, in rice and corn, a barstar gene is used as a "fertility-restoring gene" in the pollen parent (father) so as to allow the pollen in the F1 generation to restore the fertility (C. Mariani, et al., Nature 357, 384-387:1992), if an MS plant (mother) is constructed by the method with the use of a barstar gene as reported in WO96/26283, the F1 plant has plural copies of the barstar gene. In such a case, there is a possibility that the expression of the gene may be inhibited due to the gene silencing.

### SUMMARY OF THE INVENTION

The present invention provides a method for constructing a male sterile plant by using a barnase gene without resort to a barstar gene.

The present invention further provides a mutant barnase gene to be used in the above method and a process for producing the same.

### DETAILED DESCRIPTION OF THE INVENTION

In the present invention, the DNA sequence of barnase gene (R.W. Hartley, J. Mol. Biol. 202, 913-915:1988) is mutated at least in part and then the thus obtained mutant barnase gene is anther-specifically expressed in a plant so as to make the plant substantially male sterile without any substantially disadvantageous effect on the tissues other than anthers.

The mutation can be performed by a known method such as site-specific mutagenesis, deletion of a fragment by using restriction enzymes or the low fidelity PCR method. Among all, it is preferred to use the low fidelity PCR method. This method is described in detail in D. Leung, E. Chen and D. Goedda, Technique 1, 11-15:1989; Y.Z. Xiaoping and R.H. Ebright, Nucleic Acid Res. 19, 6052:1991; G.C. Rice et al., Proc. Natl. Acad. Sci. USA 89, 5467-5471: 1992 and the contents of these documents are incorporated herein by reference. By using this technique, PCR is carried out under such conditions as to induce some errors during the amplification reaction. Thus, random mutations can be introduced into the target DNA fragment.

Primers to be used in the low fidelity PCR method in the present invention are selected as in the conventional PCR method. It is preferred that each of these primers has a similar number of nucleotides as in the conventional PCR method.

The present inventors performed PCR by using a DNA containing the sequence represented by SEQ ID NO:1 as a template with the use of a combination of primer 1 (5'-CGTTCGGCTC GATGGTACCG GTTATCAACA CGTTTGA-3': SEQ ID NO: 6) and primer 2 (5'-CCTCTAGATT ATCTGATTTT TGTAAAGGTC TGATAATG-3': SEQ ID NO:7) under such conditions as to induce errors. Thus, a mutant barnase gene having the DNA sequence represented by SEQ ID NO:3 was isolated. The sequence represented by SEQ ID NO:1 employed herein is the sequence of the coding region of the barnase gene contained in the known plasmid pVE108 (WO92/ 13956). This sequence corresponds to the wild type barnase gene from which the unnecessary moiety corresponding to the secretion signal in the N-terminal side has been deleted.

It is also possible to acquire different mutant barnase genes by a similar method.

The PCR amplification product is cloned in a host (for example, *Escherichia coli*) and clones containing a mutant barnase gene is isolated. In this cloning, clones having a mutant barnase gene may be screened by assaying the RNase activity expressed by the gene. However, it is advantageous to screen the clone by the method consisting of the following two steps by taking advantage of the fact that the RNase activity of barnase affects the growth of *E*. *coli*.

In the first step, a plasmid is prepared by using the mutant barnase gene obtained above and then *E*. *coli* is transformed thereby. The growth of the *E*. *coli* transformant thus obtained will be inhibited by the activity of the mutant barnase. Based on this fact, a colony growing slowly (i.e., a small colony), compared with *E*. *coli* having a control vector free from any barnase gene, is selected. It is expected that the thus selected *E*. *coli* strain will contain the mutant barnase gene. To confirm that the inhibition of the growth of *E*. *coli* is due to the expression of the mutant barnase gene, the second step is then carried out.

In the second step, a barstar gene is employed. As described above, barstar is an inhibitory protein against barnase. In *E*. *coli* which expresses barstar, the enzymatic activity of barnase is inhibited and thus the degradation of mRNA, which otherwise takes place in the presence of barnase, can be inhibited. Therefor if, *E*. *coli* which expresses a barstar gene is transformed with a barnase gene, the growth will not be inhibited. Accordingly, it is expected that the thus obtained *E*. *coli* transformant will produce little difference in growth rate from the transformant constructed by the control plasmid which is free from any barnase gene and, therefore, these transformants will produce little difference in colony size too. Based on this principle, a plasmid is prepared from the *E*. *coli* strain selected in the first step. The plasmid is then used to transform another *E*. *coli* strain, in which the barstar gene is constitutively expressed. Thus, mutant barnase gene-containing colonies having almost the same size as the one of the *E*. *coli* transformed by the control plasmid are selected. The *E*. *coli* with the constitutive expression of the barstar gene to be used herein can be prepared by, for example, the method as will be described in Example 1 hereinafter.

The DNA sequence of the thus obtained mutant barnase gene may be analyzed by a conventional method, if necessary, to thereby examine the mutation in detail.

A preferred example of the mutant barnase gene of the present invention is one which has the DNA sequence represented by SEQ ID NO:3. Compared with the nucleotide sequence of SEQ ID NO:1 coding for the wild type activity, the nucleotide sequence encoding this gene has an insertion of "T" at the 15-position from A in the initiation codon ATG and a deletion of "A" at the 333-position. According to the normal translation manner, translation will be initiated from ATG at the 1-position of this DNA sequence represented by SEQ ID NO:3, and terminated at the ninth codon which has been converted to a termination codon as a result of the insertion of the "T" at the 15-position. It is unlikely that the thus formed oligopeptide consisting of 8 amino acids has the barnase activity. Further, there is no other ATG or GTG codon from which the translation can be initiated in the correct frame in the vicinity thereof. However, the fact that the growth rate of *E*. *coli* was restored by the barstar protein strongly suggests that the translation of the barnase protein from the gene of SEQ ID NO:3 proceeded in the correct frame. This is seemingly due to a phenomenon called "frame shift re-coding" whereby the ribosome controls the translation while automatically shifting the reading frame during the course of translation into the protein. This phenomenon, which has been reported in several genes of viruses, *E*. *coli* and animals, generally depends exclusively on an individual nucleotide sequence and neither any special protein nor translation device is required. The frame shifting efficiency during the translation varies from gene to gene, i.e., ranging from several % to 50%. In the case of the mutant barnase gene, it is considered that the efficiency of the shifting of the reading frame during the translation is not high and thus the translation product in the correct frame is formed in a smaller amount, thereby lowering the activity of the gene.

Moreover, there is a high possibility that other mutant barnase genes of which the translation efficiency is lowered due to the shifting of the reading frame, as in the case of the DNA sequence represented by SEQ ID NO:3, or some other reasons can be obtained by mutating the barnase gene by, for example, the low fidelity PCR method. Furthermore, there is a high possibility that a gene having a DNA sequence derived from the DNA sequence represented by SEQ ID NO:3 by substitution, deletion, insertion or addition of one or more nucleotides will be a mutant barnase gene which provides a lowered translation efficiency due to the shifting of the reading frame as a result of the insertion of T at the 15-position from A in the initiation codon ATG and/or the deletion of A at the 333-position, as in the DNA sequence represented by SEQ ID NO:3. It is considered that each of these genes, likewise the gene of SEQ ID NO:3, is capable of making a plant substantially male sterile when it is anther-specifically expressed in the plant. Accordingly, these genes are also included in the scope of the present invention similar to the gene of SEQ ID NO:3.

The plant which is to be male sterilized by the mutant barnase gene is not particularly limited, so long as the gene can be transferred into the plant to provide a male sterilized plant. Examples of the plants include rice, corn, tobacco, lettuce and Brassica. Among all, rice and corn are preferred.

In order to male sterilize a plant, the mutant barnase gene is expressed specifically in the anthers of the plant to inhibit the anther functions by the RNase activity of barnase. Such an anther-specific expression can be achieved by using the method described in WO92/13957. In brief, the mutant barnase gene is ligated to the downstream of an anther-specific promoter and then incorporated into plant cells with the use of an expression vector.

The incorporation may be performed by using the Agrobacterium method, the electroporation method, the particle gun method, etc.

From the transformed plant cells, a complete plant can be formed by the regeneration from a callus of the transformed plant cells in accordance with, for example, the method reported in Y. Hiel et al., Plant J. 6, 271-282:1994.

The male sterility of the thus constructed plant can be confined by the inability of the plant to fertilize unless it is pollinated from another fertilizable plant.

### Example 1: Preparation of mutant barnase gene

### Low fidelity PCR

Primer 1 (CGTTCGGCTC GATGGTACCG GTTATCAACA CGTTTGA: SEQ ID NO: 6) and Primer 2 (CCTCTAGATT ATCTGATTTT TGTAAAGGTC TGATAATG: SEQ ID NO:7) were synthesized by a DNA synthesizer (manufactured by Applied Biosystems) in accordance with the method described in S.L. Beaucage et al., Tetrahedron Lett., 22. 1859-1862:1982. Known plasmid pVE108 (WO92/13956) was used as a template and PCR was carried out with the use of the combination of Primers 1 and 2. SEQ ID NO:1 shows the sequence of the coding region of the barnase gene which is contained in pVE108. The reaction was performed for 50 cycles with each cycle consisting of 1 minute at 94°C, 1 minute at 57°C and 1 minute at 72°C in 10 mM Tris-HCl (pH 9.5), 50 mM KCl, 2 mM MgCl₂, and 1 mM each of dNTP, 10 ng of the template DNA and 0.5 U of Taq DNA polymerase.

After the completion of the reaction, the amplification product was separated by electrophoreses on agarose gel (2% SeaKem GTG agarose, 1xTAE) and purified by the DEAE-cellulose method (M. Muramatsu, Labo-manual Idenshikogaku (Gene Engineering Labo-Manual), Maruzen, pp.111:1988). By using the purified product thus obtained as a template. PCR was performed again under the conditions as defined above.

### Ligation of mutated barnase gene fragment into plasmid vector

The reaction product from the above step was digested with SacI and XbaI in a conventional manner and then purified by agarose gel electrophoresis to give an "insertion fragment". A plasmid to be used for transferring this insertion fragment into *E*. *coli* can be appropriately selected. For example, use may be made of plasmid pHM1 therefor. The plasmid pHM1 was constructed as follows. Plasmid pBR322 was cleaved at the EcoRI site and blunt-ended by using T4 DNA polymerase (manufactured by Takara Shuzo). Separately, lacZ expression cassette (322 bp) was excised from pUC18 with PvuII and the ends thereof were blunted. The cassette was integrated into the restriction site of the blunt-ended plasmid pBR 322 to give plasmid pHM1.

Plasmid pHM1 was digested with SacI and XbaI followed by dephosphorylation with calf intestine alkaline phosphatase (manufactured by Takara Shuzo) to give a restriction enzyme-treated plasmid fragment. Into this restriction enzyme-treated plasmid pHM1, the above "insertion fragment" containing a barnase gene was ligated by using Takara Ligation Kit ver.1 (manufactured by Takara Shuzo).

### Introduction into E. coli and selection of barnase-active clone

The *E*. *coli* having the barnase gene introduced thereinto may be selected by, for example, the following method. Since mRNA is degraded in cells by the barnase activity, the synthesis of protein is suppressed and, in its turn, the growth of *E*. *coli* is inhibited. Thus, the *E*. *coli* transformed by the barnase gene can be selected by taking advantage of the fact that the desired transformant colony has a smaller size than the *E*. *coli* colony transformed by the control plasmid free from the mutant barnase gene. When the wild type barnase or a mutant barnase still maintaining the activity comparable thereto is integrated into pHM1, *E*. *coli* cannot form any colony. Thus, clones having a sufficiently weakened barnase activity can be exclusively selected.

Thus, the plasmid having the mutant barnase gene ligated thereto was precipitated from ethanol and then introduced into *E*. *coli* LE392 strain by the electroporation method with the use of GenePulser (BioRad) in accordance with the manufacture's instruction. Also, the control plasmid free from mutant barnase was introduced into the *E*. *coli* LE392 strain in the same manner.

Next, these *E*. *coli* transformants were plated onto an LB agar medium containing tetracycline (25 µg/ml) and incubated at room temperature (25°C) for 72 hours. Then colonies of a smaller size than the colony of *E*. *coli* having the control plasmid pHM1 introduced thereinto were screened as colonies of the *E*. *coli* transformants having the mutant barnase transferred thereinto (Table 1, A).

### Selection of mutant barnase clone by barstar gene

As described above, barstar is an enzyme acting as an antagonist to barnase. In *E*. *coli* wherein barstar is expressed, the enzymatic activity of barnase is inhibited and thus the degradation of mRNA, which otherwise takes place in the presence of barnase, can be inhibited. Therefore, if *E*. *coli* expressing barstar is transformed by a plasmid containing a barnase gene, the growth thereof will not be inhibited. Accordingly, it is expected that the thus obtained *E*. *coli* transformant will show little difference in growth rate from the transformant constructed by using the control plasmid free from a barnase gene and, therefore, these transformants will show little difference in colony size too.

An *E*. *coli* transformant which expressed barstar was constructed in the following manner. A barstar gene described in R.W. Hartley, J. Mol. Biol. 202, 913-915:1998 was excised with HindIII and XbaI and ligated in frame to a tac promoter (de Boer et al., Proc. Natl. Acad. Sci. USA 80, 21-25:1983). The gene, together with a chloramphenicol tolerance gene (N.K. Alton and D. Vapnek, Nature 282, 864-869:1979), was ligated into the defective transposon which lacked the transferase in a vector described in Herrero et al., J. Bacteriol. 172, 6557-6567:1990. Subsequently, the thus obtained plasmid was introduced into *E*. *coli* MC1061 strain thereby to transfer the transposon, which contained the barstar gene cassette, into the chromosome of the *E*. *coli*. It was confirmed based on the chloramphenicol tolerance that this *E*. *coli* was able to maintain the barstar gene cassette in a stable state. Since the *E*. *coli* MC1061 strain lacked the lacI gene, the tac promoter was continuously active and thus the barstar gene was constitutively expressed.

The plasmid containing the mutant barnase gene as the insertion fragment and the control plasmid free from the same were each introduced into the above *E*. *coli* transformant by the electroporation method. Then the *E*. *coli* transformants were plated onto an LB agar medium containing IPTG (1mM) and tetracycline (25 µg/ml) and incubated at 25°C for 72 hours. Among the colonies of the *E*. *coli* transformed by the mutant barnase gene, a colony being comparable in size to the colony of the *E*. *coli* having the control plasmid pHM1, which had been simultaneously plate-cultured, was screened and named "#4-31" (Table 1, B).

**Table 1**

| Screened clone and colony size (mm) | | |
|---|---|---|
| | Screened clone | pHM1 (control) |
| A: *E*. *coli* LE392*¹ | 1.77 ± 0.33 | 2.65 ± 0.47 |
| B: *E*. *coli* with barstar gene expression*² | 1.39 ± 0.10 | 1.55 ± 0.08 |

| | | |
|---|---|---|
| *1: Colony size after incubation on the LB agar plate containing 25 µg/mL of tetracycline at 28°C for 48 hours. | | |
| *2: Colony size after incubation on the LB agar plate containing 25 µg/mL of tetracycline at 28°C for 24 hours. | | |

### Example 2: Determination of nucleotide sequence of mutant barnase gene

From the *E*. *coli* screened in Example 1, a cloned mutant barnase gene appropriate for the object of the present invention was prepared. First, the clone #4-31 screened in Example 1 was propagated. Next, the mutant barnase gene fragment in the clone #4-31 was excised with KpnI and XbaI and ligated into the KpnI, XbaI-site of pUC119 having been cleaved similarly with KpnI and XbaI. After amplifying the obtained plasmid by using *E*. *coli*, a reaction was performed by the cycle sequence method with the use of Taq polymerase (Taq Dye Terminator Cycle Sequencing Kit, manufactured by Applied Biosystems Inc.) in accordance with the manufacturer's protocol. Subsequently, the sequence was analyzed with a DNA Sequencer (Model 373A, manufactured by Applied Biosytems Inc.), thereby yielding the sequence represented by SEQ ID NO:3. Compared with the DNA sequence of the wild type barnase gene, this sequence had an insertion of T at the 15-position from A of the initiation codon ATG and a deletion of A at the 333-position.

### Example 3: Construction of male sterile rice by using attenuated mutant barnase gene

The attenuated mutant barnase gene integrated into pUC119 as described above was cleaved with restriction enzymes XbaI and KpnI and thus the plasmid vector pTS431 represented by SEQ ID NO:5 was constructed. Although this plasmid pTS431 differs from the known plasmid pVE108 (PCT International Publication WO92/13956) in the following points, it is substantially equivalent thereto except for the moieties of the anther-specific promoter and the mutant barnase.
(1) In the plasmid vector pTS431 according to the present invention, the barnase gene (SEQ ID NO:1) in the known plasmid pVE108 has been converted into the mutant barnase gene (SEQ ID NO:3).
(2) A tobacco-origin anther specific promoter is used in the known plasmid pVE108, while the anther specific promoter of rice E1 gene (PCT International Publication WO92/13956) is used in the plasmid vector pTS431 according to the present invention.
(3) In the present invention, a 35S3 promoter (EP 0344029) of 1376 bp, which is not used in the plasmid pVE108, is employed upstream of the barnase gene.
(4) In the present invention, a sequence originating in the downstream of Agrobacterium T-DNA gene 7 excised from pJD884 (PCT International Publication WO93/09218) is employed whereas such a sequence is not used in the plasmid pVE108.
(5) In the present invention, the region corresponding to lacZ has been deleted from the pUC19-origin moiety. In addition a plasmid (pTS172) having the wild type barnase gene integrated therein is represented by SEQ ID NO:4.

The fragment of about 4.5 kbp was excised with a restriction enzyme EcoRI respectively from pTS431 (a plasmid having the mutant barnase gene transferred thereinto; SEQ ID NO:5) and pTS172 (a plasmid having the barnase gene transferred thereinto; SEQ ID NO:4) and inserted into the EcoRI-site of the intermediate vector pSB11 (T. Komari et al., Plant J. 10(1), 165-174:1996), and further, the T-DNA region thereof was integrated into the acceptor vector pSB1 (T. Komari et al., Plant J. 10(1). 165-174:1996) by way of homologous recombination. By using *Agrobacterium tumefaciens* LBA4404 having the obtained recombinant plasmids (pSB1431 and pSB1172 respectively), rice (variety: Asanohikari) was transformed. While the transformation was performed basically in accordance with the method of Hiei et al. (Plant J. 6(2), 271-282:1994). phosphinothricine (concentration: 10 mg/L) was employed for screening transformants since the thus constructed male sterility genes contained bar gene encoding phosphinithricine acetyl transferase as a selective marker. Phosphinothricine facilitates selection of calluses having the gene transferred thereinto.

A comparison of the rice transformant obtained with the use of the wild type barnase gene with the one obtained with the use of the mutant barnase gene indicates that the transfer of the mutant barnase gene gave rise to a remarkable improvement in the transformation efficiency and an increase in the rate of morphologically normal male sterility transformants, as shown in Table 2.

**Table 2**

| Transformation efficiency | | | | |
|---|---|---|---|---|
| | No. of infected calluses | No. of regenerated calluses | No. of PCR-positive lines*¹ | No. of morphologically normal male sterile lines |
| pSB1172 (control) | 2838 | 83 | 52/83 | 9/52(17.3%) |
| pSB1431 (present invention) | 787 | 69 | 43/45*² | 27/28*³(96.4%) |

| | | | | |
|---|---|---|---|---|
| *1: detection of barnase gene fraction by PCR. | | | | |
| *2: examined only 45 lines among 69. | | | | |
| *3: examined only 28 lines among 43. | | | | |

### Effects of the Invention

In the present invention, a barnase gene having a weakened effect is constructed via mutation. A male sterility gene comprising the mutant barnase gene can be introduced into a plant to successfully produce a male sterile plant free from any unfavorable characteristic at a high efficiency with the use of a single gene without resort to the use of a barstar gene.

## Claims

1. A mutant barnase gene having one or some mutations at least in part of the DNA sequence of barnase gene, wherein said mutant barnase gene is capable, when anther-specifically expressed in a plant, of making said plant substantially male sterile without exerting any substantially disadvantageous effect on the tissues except for the anthers.

2. The gene as claimed in Claim 1, wherein said mutation is a mutation causing frame shift re-coding.

3. A gene which is derived from a DNA sequence encoding the same amino acid sequence as shown in SEQ ID NO:1 by substitution, deletion, insertion or addition of one to several nucleotides in said DNA sequence, wherein said gene is capable of encoding a protein which makes a plant substantially male sterile when expressed anther-specifically in said plant.

4. A gene comprising a DNA sequence which is derived from a DNA sequence encoding the same amino acid sequence as shown in SEQ ID NO:1 by substitution, deletion, insertion or addition of one to several nucleotides in the latter DNA sequence, and a promoter located upstream of said DNA sequence for allowing an anther-specific expression, wherein said gene is capable of making a plant substantially male sterile when introduced into the genome of said plant.

5. A gene represented by SEQ ID NO:3 which encodes a protein capable of making a plant substantially male sterile when expressed anther-specifically in said plant.

6. A gene comprising the sequence represented by SEQ ID NO:3 and a promoter located upstream of said sequence for allowing an anther-specific expression, wherein said gene is capable of making a plant substantially male sterile when introduced into the genome of said plant.

7. A recombinant vector which contains a gene as claimed in any of Claims 1 to 6 and expresses said gene in a host plant.

8. A method of making a plant male sterile which comprises transforming said plant by a mutant barnase gene as claimed in any of Claims 1 to 6 and allowing said mutant barnase gene to be expressed anther-specifically.

9. The method as claimed in Claim 8, wherein said plant is transformed by said mutant barnase gene by integrating said gene into the genome of said plant.

10. A transgenic plant wherein a gene as claimed in any of Claims 1 to 6 has been introduced.
